# FASCICULE DE BREVET EUROPEEN

(11) **EP 2 857 023 B1**
(45) Date de publication et mention de la délivrance du brevet: **20.04.2016**
(21) Numéro de dépôt: 14187588.0
(22) Date de dépôt: 03.10.2014
(51) Int. Cl.: A61K 31/7042, A61P 43/00

(54) **Utilisation de l'odiparcil dans le traitement d'une mucopolysaccharidose**
Verwendung von Odiparcil zur Behandlung einer Mukopolysaccharidose
Use of odiparcil in the treatment of mucopolysaccharidosis

(30) Priorité: 04.10.2013 FR 1359657
(43) Date de publication de la demande: 08.04.2015
(60) Demande divisionnaire: 16159903.0
(73) Titulaire: INVENTIVA, 21121 Daix (FR)
(72) Inventeur: Masson, Philippe, 21121 HAUTEVILLE-LES-DIJON (FR); Junien, Jean-Louis, 92310 SEVRES (FR)
(74) Mandataire: Nevant, Marc

(56) Documents cités:
- EP-A1- 0 421 829
- MYERS ALAN L ET AL: "Characterization of total plasma glycosaminoglycan levels in healthy volunteers following oral administration of a novel antithrombotic odiparcil with aspirin or enoxaparin", JOURNAL OF CLINICAL PHARMACOLOGY, vol. 48, no. 10, octobre 2008 (2008-10), pages 1158-1170, XP002719465, ISSN: 0091-2700

## Description

La présente invention concerne l'utilisation de l'odiparcil, ou d'une composition pharmaceutique contenant ce composé, dans le traitement d'une mucopolysaccharidose, et notamment de la maladie de Maroteaux-Lamy.

### Arrière-plan technique de l'invention

Les mucopolysaccharidoses (MPS) sont des maladies génétiques dégénératives liées à un déficit enzymatique. En particulier, les MPS sont causées par la déficience ou l'inactivité des enzymes lysosomales qui catalysent le métabolisme progressif de molécules de sucre complexes appelées glycosaminoglycanes (GAG). Ces déficiences enzymatiques engendrent une accumulation de GAG dans les cellules, les tissus et, en particulier, les lysosomes cellulaires des sujets affectés, entraînant des dommages cellulaires permanents et progressifs qui affectent l'apparence, les capacités physiques, le fonctionnement des organes, et, dans la plupart des cas, le développement mental des sujets atteints.

Onze déficits enzymatiques distincts ont été identifiés, correspondant à sept catégories cliniques distinctes de MPS. Chaque MPS est caractérisée par une déficience ou inactivité d'une ou plusieurs enzymes qui dégradent les mucopolysaccharides que sont l'héparanesulfate, le dermatanesulfate, le chondroïtinesulfate, le kératanesulfate.

La mucopolysaccharidose de type III (MPS III) ou maladie de Sanfilippo est une maladie de surcharge lysosomale, du groupe des mucopolysaccharidoses, caractérisée par une dégradation intellectuelle sévère et rapide. Les premiers symptômes apparaissent entre 2 et 6 ans : troubles du comportement (hyperkinésie, agressivité) et dégradation intellectuelle, troubles du sommeil avec des signes dysmorphiques très modérés. L'atteinte neurologique devient plus marquée vers l'âge de 10 ans avec la perte des acquisitions psychomotrices et de la communication avec l'entourage. Une épilepsie survient souvent après l'âge de 10 ans. La maladie est due à la présence d'héparanesulfate non dégradé en raison du déficit de l'une ou l'autre des 4 enzymes nécessaires à son catabolisme, responsable de l'un des 4 types de MPS III : type IIIA (héparane sulfamidase), type IIIB (alpha-N-acétylglucosaminidase), type IIIC (acétylCoA : alpha-glucosaminide-N-acétyltransférase), et type IIID (N-acétylglucosamine-6-sulfate sulfatase). Il n'existe pas à ce jour de traitement efficace de cette maladie.

La mucopolysaccharidose de type VI (MPS VI) ou maladie de Maroteaux-Lamy est une maladie de surcharge lysosomale, du groupe des mucopolysaccharidoses, caractérisée par une atteinte somatique sévère et une absence de régression psycho-intellectuelle. La prévalence de cette mucopolysaccharidose rare est comprise entre 1/250 000 et 1/600 000 naissances. Dans les formes sévères, les premières manifestations cliniques apparaissent entre 6 et 24 mois et s'accentuent progressivement : dysmorphie faciale (macroglossie, bouche constamment entrouverte, traits épais), limitations articulaires, dysostose multiple très sévère (platyspondylie, cyphose, scoliose, pectus carinatum, genu valgum, déformation des os longs), petite taille (inférieure à 1,10 m), hépatomégalie, atteinte valvulaire cardiaque, cardiomyopathie, surdité, opacités cornéennes. Le développement intellectuel est habituellement normal ou quasi normal mais les atteintes auditives et ophtalmologiques peuvent engendrer des difficultés d'apprentissage. Les symptômes et la sévérité de la maladie varient considérablement d'un patient à l'autre et il existe également des formes intermédiaires, voire très modérées (dysplasie spondylo-épiphyso-métaphysaire associée à des atteintes cardio-respiratoires). Comme les autres mucopolysaccharidoses, la maladie de Maroteaux-Lamy est lié au déficit d'une enzyme du métabolisme des mucopolysaccharides, en l'occurrence la N-acétylgalactosamine-4-sulfatase (également appelée arylsulfatase B). Cette enzyme métabolise le groupement sulfate du dermatanesulfate (Neufeld et al. : « The mucopolysaccharidoses » The Metabolic Basis of Inherited Diseases, eds. Scriver et al, New York, McGraw-Hill, 1989, p. 1565-1587). Ce déficit enzymatique bloque la dégradation progressive de dermatanesulfate, ce qui entraîne une accumulation de dermatanesulfate dans les lysosomes des tissus surchargés. A ce jour, il existe un seul médicament autorisé pour le traitement de cette maladie : le Naglazyme® (galsulfase recombinante humaine) dont le coût est extrêmement élevé (aux Etats-Unis, il est de l'ordre de 350 000 $ par an). Une alternative à ce traitement est l'allogreffe de moelle osseuse.

La mucopolysaccharidose de type VII (MPS VII) ou maladie de Sly est une maladie de surcharge lysosomale, très rare, du groupe des mucopolysaccharidoses. La symptomatologie est extrêmement hétérogène : formes anténatales (anasarque foetoplacentaire non immune), formes néonatales sévères (avec dysmorphie, hernies, hépatosplénomégalie, pieds bots, dysostose, hypotonie importante et troubles neurologiques évoluant vers un retard statural et un déficit intellectuel profond en cas de survie) et formes très modérées découvertes à l'adolescence voire à l'âge adulte (cyphose thoracique). La maladie est due au déficit en bêta-D-glucuronidase, responsable de l'accumulation dans les lysosomes de divers glycosaminoglycanes : dermatanesulfate, héparanesulfate, et chondroïtinesulfate. Il n'existe pas à ce jour de traitement efficace de cette maladie.

Il existe donc clairement un besoin de mettre à disposition des sujets atteints de MPS de type III, VI et VII un traitement médicamenteux, et dans le cas de la MPS de type VI, un traitement alternatif non issu des biotechnologies.
L'Odiparcil (4-méthyl-2-oxo-2H-1-benzopyran-7-yl-5-thio-β-D-xylopyranoside ; CAS 137215-12-4) appartient à la famille des thioxylosides. Ce composé, décrit dans la demande de brevet EP-A-0 421 829, répond à la formule :

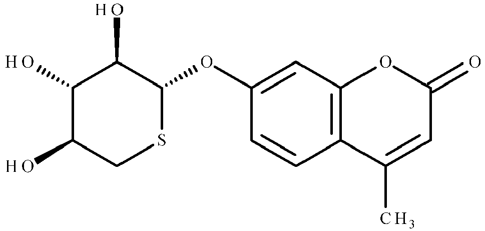

Ce composé a fait l'objet d'un développement clinique (phases 1 et 2) dans le traitement de la thrombose à la fin des années 90 et au début des années 2000. Son mécanisme d'action peut être résumé de la manière suivante : l'Odiparcil se comporte comme un substrat pour une enzyme, la GT1 (galactosyl transférase 1), qui initie la synthèse des chaines des GAG vers la voie des dermatanesulfate/chondroïtinesulfate. Ces GAG sont des constituants de la cellule en tant que protéoglycanes (quand ils sont liés aux protéines sur une sérine et un premier sucre qui est le xylose) et sont également sécrétés dans le milieu extracellulaire. Ils ont des rôles variés allant du contrôle de la coagulation (héparine/héparane et dermatanesulfate secrétés dans la circulation) à la régulation de facteurs de croissances (bêtaglycane).

Il a maintenant été constaté, et c'est l'objet de la présente invention, que l'Odiparcil permet d'augmenter la synthèse des GAG totaux au niveau extracellulaire et, par là même, va contribuer à diminuer la charge intracellulaire des GAG en jouant un rôle de « leurre », rendant plus efficace l'activité résiduelle de la N-acétylgalactosamine-4-sulfatase. Il est ainsi possible d'envisager le traitement des MPS de type III, VI et VII du fait de la diminution de l'accumulation de GAG au niveau intracellulaire.

### Objet de l'invention

Selon un premier aspect, l'invention concerne l'Odiparcil pour son utilisation dans le traitement de la mucopolysaccharidose de type III, VI ou VII, et notamment dans le traitement de la MPS de type VI (également appelée maladie de Maroteaux-Lamy). L'Odiparcil et son procédé d'obtention sont décrits dans la demande de brevet EP-A-0 421 829.

Dans le contexte de la présente invention, le terme "Odiparcil" désigne la forme « β-D-xylopyranoside ».

Dans un mode de réalisation, l'Odiparcil utilisé dans le cadre de l'invention a au moins 60%, de préférence au moins 70%, au moins 80%, au moins 90%, au moins 95%, au moins 98% ou au moins 99% de configuration D. Dans ce mode de réalisation, l'Odiparcil est de préférence sous forme d'anomère β.

Dans un autre mode de réalisation, l'Odiparcil utilisé dans le cadre de l'invention est au moins 60%, de préférence au moins 70%, au moins 80%, au moins 90%, au moins 95%, au moins 98% ou au moins 99% sous la forme d'anomère β.

De manière avantageuse, l'Odiparcil est administré à raison d'environ 100 mg à environ 5000 mg par jour. Par exemple, on administre environ 100, 250, 300, 375, 400, 500, 750, 800, 1000, 1500, 2000, 3000, 4000 ou 5000 mg d'Odiparcil quotidiennement.

Dans un mode de réalisation, on administre quotidiennement au moins environ 0,1 mg à environ 70 mg d'Odiparcil par kg de poids corporel du patient. Par exemple, on administre quotidiennement au moins environ 1 ou 2 mg, à environ 5, 10, 15, 20, 25, 30, 35, 40, 45, 50, 55, 60, 65 ou 70 mg d'Odiparcil par kg de poids corporel du patient.

Dans un mode de réalisation, l'Odiparcil est administré une à deux fois par jour (par exemple, toutes les 10 à 12 heures). Ainsi, les doses journalières mentionnées ci-dessus peuvent être réparties pour une administration biquotidienne (bid), par exemple une dose journalière de 1000 mg sera administrée à raison de deux doses de 500 mg chacune. Il est entendu que chaque dose peut être constituée d'une ou plusieurs formes pharmaceutiques, par exemple une dose de 500 mg peut être constituée de deux formes pharmaceutiques de 250 mg chacune.

Dans un mode de réalisation, l'Odiparcil est administré à jeun (c.à.d. l'estomac vide, par exemple au moins 1h avant de manger ou plus de 2h après manger). Dans un autre mode de réalisation, l'Odiparcil est administré lors d'une prise de nourriture (c.à.d. en même temps que ou juste avant la prise d'un repas, par exemple environ 20 à 30 min avant un repas ou dans les 5 min qui suivent la fin d'un repas).

Dans un mode de réalisation, l'Odiparcil est formulé dans une composition pharmaceutique contenant un ou plusieurs excipients pharmaceutiquement acceptables, selon des techniques bien connues de l'homme du métier, comme par exemple celles décrites dans l'ouvrage « Remington, The Science and Practice of Pharmacy, 21st Edition, Lippincott Williams & Wilkins, 2006 ».

Ainsi, selon un second aspect, l'invention concerne une composition pharmaceutique contenant l'Odiparcil, et un ou plusieurs excipients pharmaceutiquement acceptables, pour son utilisation dans le traitement de la mucopolysaccharidose de type III, VI ou VII, notamment le traitement de la maladie de Maroteaux-Lamy.

La composition pharmaceutique peut se présenter sous toute forme adaptée à la voie d'administration désirée. Cette administration peut être per os, linguale, sublinguale, buccale, rectale, topique, intraveineuse, intra-artérielle, sous-cutanée, intranasale, transdermique, intramusculaire ou intrapéritonéale.

Dans un mode de réalisation, la composition pharmaceutique contient environ 100 à 1000 mg d'Odiparcil, par exemple 100, 125, 150, 250, 375, 400, 500 ou 1000 mg d'Odiparcil.

Dans un mode de réalisation, la composition pharmaceutique est administrée par voie injectable, et comprend un véhicule qui est typiquement une solution aqueuse stérile contenant parfois, en plus de l'eau, un ou plusieurs ingrédients tels que des sucres, des conservateurs, des sels, des tampons etc. Les suspensions injectables peuvent comprendre un agent de suspension et un véhicule liquide donné.

Dans un mode de réalisation, la composition pharmaceutique est administrée par voie orale. Des formes galéniques orales appropriées comprennent les formulations solides et liquides. Lorsque la composition pharmaceutique est une formulation solide (comme, par exemple, des gélules, des comprimés, des poudres sèches), des excipients utiles incluent notamment les diluants, les lubrifiants, les liants, les agents désintégrants, les charges etc. Les formulations solides peuvent être enrobées ou non ; lorsqu'elles le sont, l'enrobage peut être entérique ou non-entérique. Lorsque la composition pharmaceutique est une formulation liquide (comme par exemple un élixir ou un sirop), les excipients utiles incluent par exemple l'eau, les glycols, une solution salée, les alcools, les agents aromatisants etc.

De manière avantageuse, la composition pharmaceutique est un comprimé. Une telle composition est préparée en une ou plusieurs étapes, comprenant le mélange des différents constituants jusqu'à l'obtention d'un mélange homogène, et la compression du mélange pour obtenir un comprimé. Dans un mode de réalisation, la composition est préparée par un procédé de granulation humide, technique bien connue de l'homme du métier. Par exemple, l'Odiparcil, tout ou partie du diluant, le liant, et une quantité suffisante de fluide de granulation (comme l'eau) sont combinés, granulés, séchés et broyés pour former des granulés. Les granulés sont ensuite combinés ou non avec le reste des constituants et le mélange est comprimé. Les comprimés comprennent avantageusement environ 5% à environ 90% d'Odiparcil, par rapport au poids total du comprimé.

Selon un troisième aspect, l'invention concerne une méthode de traitement de la mucopolysaccharidose de type III, VI ou VII, notamment de la maladie de Maroteaux-Lamy, qui consiste à administrer à un sujet en ayant besoin une quantité thérapeutiquement efficace d'Odiparcil ou d'une composition pharmaceutique contenant ce composé. Dans un mode de réalisation, la posologie journalière d'Odiparcil et la composition pharmaceutique sont telles que définies ci-dessus.

L'invention est illustrée par la partie expérimentale ci-dessous.

### Activité pharmacologique

### 1. Résultats obtenus sur cellules en culture

### 1.1. Cellules endothéliales d'aorte bovine

Des cellules endothéliales d'aorte bovine (ECACC 92010601), cultivées en plaques de 6 puits, sont incubées 24 h en présence de sulfate de sodium ³⁵S (10 µci/mL) et d'Odiparcil solubilisé dans le DMSO à différentes concentrations (1-10 µM ; 0,1% DMSO final). Les surnageants de culture sont récupérés et les tapis cellulaires rincés par du tampon phosphate (PBS). Les surnageants de culture et les solutions de rinçage sont regroupés dans des tubes. Une solution de dermatanesulfate non marqué (200 µg) est ensuite ajoutée afin de servir d'entraineur. Le ³⁵S non incorporé est ensuite éliminé par gel filtration sur colonnes de Sephadex G25, les GAG étant élués dans la fraction d'exclusion (V0) de la colonne. A l'éluant, une solution de chlorure de cétylpyridinium (0,1% final) est ajoutée afin de précipiter les GAG pendant 24 h à température ambiante. Les échantillons sont ensuite centrifugés et le surnageant éliminé. Le précipité obtenu est remis en solution dans du chlorure de magnésium 2 M et les GAG sont précipités par 5 volumes d'éthanol à 95%. Après centrifugation, les précipités alcooliques sont remis en solution dans du chlorure de sodium à 0,9% puis la radioactivité est mesurée sur une fraction aliquote après ajout de liquide scintillant dans des fioles de comptage.

Afin de typer les GAG produits dans les surnageants de cellules en culture, les précipités alcooliques remis en solution sont traités par la chondroïtinase ABC (*Proteus vulgaris*) à raison de 0,5 mU/µL, pendant 3 h à 37°C. Après inactivation de l'enzyme 3 min à 100°C, les GAG non digérés sont précipités par 5 volumes d'éthanol à 95%, pendant 1 nuit à 4°C. Après centrifugation les précipités alcooliques sont remis en solution dans du chlorure de sodium à 0,9% puis la radioactivité est mesurée sur une fraction aliquote après ajout de liquide scintillant dans des fioles de comptage.

Pour les GAG de type héparanesulfate, ceux-ci sont traités par l'héparinase II (*Flavobacterium heparinum*) à raison de 4 mU/µL, pendant 12 h à 30°C. Après inactivation de l'enzyme 3 min à 100°C, les GAG non digérés sont précipités par 5 volumes d'éthanol à 95%, pendant 1 nuit à 4°C. Après centrifugation les précipités alcooliques sont remis en solution dans du chlorure de sodium à 0,9% puis la radioactivité est mesurée sur une fraction aliquote après ajout de liquide scintillant dans des fioles de comptage.

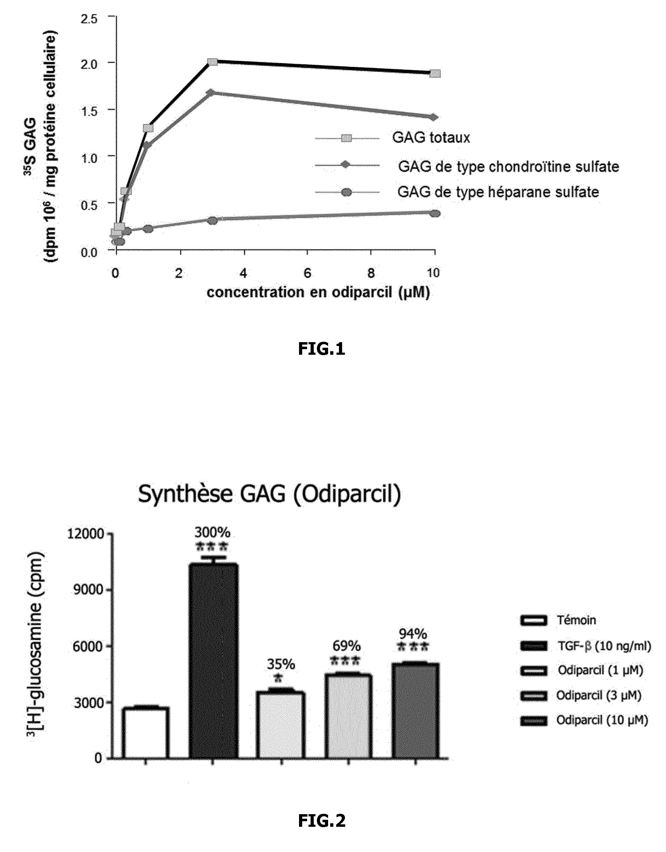

Comme on peut le voir sur la figure 1, l'Odiparcil augmente de façon dose-dépendante le taux de GAG marqués au ³⁵S dans le surnageant de culture de cellules endothéliales aortiques bovines.

De plus, les digestions enzymatiques indiquent que les GAG synthétisés par les cellules en culture sont majoritairement de type chondroïtinesulfate.

### 1.2. Fibroblastes humains

Des fibroblastes dermiques humains normaux (BIOAlternatives PF2) sont cultivés en plaques de 96 puits pendant 24 h. Le milieu de culture est ensuite remplacé par du milieu de culture contenant ou non (témoin) l'Odiparcil à différentes concentrations (1 µM, 3 µM, 10 µM) ou la référence TGF-β à 10 ng/mL (témoin positif) puis les cellules sont incubées pendant 72 h avec ajout du marqueur radioactif ³H-glucosamine pour l'évaluation de la synthèse des GAG totaux. A la fin de l'incubation, un tampon chaotropique est ajouté dans les puits des plaques de culture afin de lyser les fibroblastes. Les GAG totaux des lysats cellulaires sont ensuite purifiés par chromatographie d'échange d'ions (colonne Q-Sepharose). La radioactivité incorporée dans les fractions anioniques est mesurée par scintillation liquide.

Comme on peut le voir sur la figure 2, l'Odiparcil stimule de façon dose-dépendante la synthèse de GAG totaux par des fibroblastes dermiques humains (+94% à 10 µM). Les données ont été analysées statistiquement par analyse de variance à un facteur suivie par un test de Dunnett (* p<0,05 vs témoin ; ** p<0,01 vs témoin ; *** p<0,001 vs témoin).

### 2. Résultats obtenus in vivo chez le lapin après administration orale.

L'Odiparcil est administré par voie orale à la dose de 400 mg/kg chez le lapin New Zealand. 4 h après l'administration les animaux sont anesthésiés, et le sang prélevé sur tubes citratés après cathétérisation de l'artère carotide. Après centrifugation, le plasma est prélevé et congelé. Les GAG plasmatiques sont isolés après digestion des protéines par la Pronase E, pendant 48 h à 50°C. Les protéines et les résidus protéiques sont précipités par ajout d'acide trichloracétique et incubation pendant 1 nuit à 4°C. Après centrifugation les surnageants sont recueillis, puis dialysés contre 100 volumes de tampon phosphate, pendant 48 h à 4°C. Une solution de chlorure de cétylpyridinium (0,1% final) est ajoutée aux dialysats afin de précipiter les GAG pendant 24 h à température ambiante. Les échantillons sont ensuite centrifugés et le surnageant éliminé. Le précipité obtenu est remis en solution dans du chlorure de sodium 2M et les GAG sont précipités par 5 volumes d'éthanol à 95%. Après centrifugation, les précipités alcooliques sont remis en solution dans du chlorure de sodium à 0,9% et dessalés sur colonne de Sephadex G25 (PD10).

Les GAG plasmatiques extraits sont quantifiés par dosage de la teneur en acide uronique, méthode au carbazole de Bitter et Muir modifiée. L'analyse qualitative des extraits de GAG plasmatiques est réalisée par HPLC des disaccharides obtenus après digestion enzymatique par la chrondroïtinase ABC de *Proteus vulgaris* et la chrondroïtinase AC d'*Arthrobacter aurescens.*

Le tableau ci-dessous montre que le traitement des animaux par l'Odiparcil à la dose de 400 mg/kg augmente d'un facteur 5 le taux de GAG plasmatiques (mesuré par le contenu en acide uronique) comparativement aux animaux contrôles. D'un point de vue qualitatif les GAG de type chondroïtine voient leur composante galactosamine 6-sulfate augmentée, ainsi que la composante dermatanesulfate (chondroïtine B), mesurée par les disaccharides galactosamine 4-sulfate (Δdi-4S DS).

| | µg UA/ml plasma | Δdi-0S (%) | Δdi-4S (%) | Δdi-6S (%) | Δdi-UA2S (%) | Δdi-4S DS (%) |
|---|---|---|---|---|---|---|
| Témoin | 2,1 | 51,1 | 45,8 | 3,1 | 0 | 0 |
| Odiparcil | 11,4 | 18,6 | 26 | 30,8 | 4,1 | 20,5 |

| | | | | | | |
|---|---|---|---|---|---|---|
| UA: Acide Uronique Δdi-0S : disaccharides non sulfatés Δdi-4S : disaccharides-4-sulfatés Δdi-6S : disaccharides-6-sulfatés (composante galactosamine 6-sulfate) Δdi-UA2S : dissacharides-2UA-sulfatés Δdi-4S DS : disaccharides-4-sulfatés (composante dermatanesulfate) | | | | | | |

Ces résultats démontrent que l'Odiparcil a la capacité d'augmenter la synthèse des GAG totaux (fibroblastes humains), d'augmenter la concentration des GAG extracellulaires de type chondroïtine (cellules endothéliales d'aorte bovine) et d'augmenter la synthèse de GAG plasmatiques, notamment pour les GAG de type chondroïtine. Etant entendu que les MPS de type III, VI et VII sont caractérisées par une accumulation de GAG intracellulaires, ces résultats indiquent que l'Odiparcil a la capacité de diminuer la charge en GAG intracellulaires et donc d'avoir des effets bénéfiques dans le traitement desdites MPS.

### Exemple de formulation galénique

Comprimé obtenu par un procédé de granulation humide contenant (% en poids) :

| | |
|---|---|
| Odiparcil | 90% |
| Cellulose microcrystalline (NF or Ph Eur) | 7% |
| Povidone ou Polyvinylpyrrolidone (USP ou Ph Eur) | 3% |
| Eau (USP ou Ph Eur) | qs pour granulation humide |

## Revendications

1. Odiparcil pour son utilisation dans le traitement d'une mucopolysaccharidose de type III, VI ou VII.

2. Odiparcil pour l'utilisation selon la revendication 1, qui est destiné à être administré à raison d'environ 100 mg à environ 5000 mg par jour.

3. Odiparcil pour l'utilisation selon la revendication 1 ou la revendication 2, qui est destiné à être administré par voie orale.

4. Odiparcil pour l'utilisation selon la revendication 3, qui est destiné à être administré avec de la nourriture.

5. Odiparcil pour l'utilisation selon l'une des revendications 1 à 4 dans le traitement de la maladie de Maroteaux-Lamy.

6. Composition pharmaceutique contenant de l'odiparcil et un ou plusieurs excipients pharmaceutiquement acceptables, pour son utilisation dans le traitement de la mucopolysaccharidose de type III, VI ou VII.

7. Composition pharmaceutique pour l'utilisation selon la revendication 6, qui contient de 100 mg à 1000 mg d'odiparcil.

8. Composition pharmaceutique pour l'utilisation selon la revendication 6 ou la revendication 7, qui est une forme galénique orale, de préférence une formulation solide.

9. Composition pharmaceutique pour l'utilisation selon la revendication 8, qui est un comprimé.

10. Composition pharmaceutique pour l'utilisation selon l'une des revendications 6 à 9 dans le traitement de la maladie de Maroteaux-Lamy.

## Patentansprüche

1. Odiparcil zur Verwendung bei der Behandlung einer Mucopolysaccharidose des Typs III, VI oder VII.

2. Odiparcil zur Verwendung nach Anspruch 1, das für eine Verabreichung von ungefähr 100 mg bis ungefähr 5000 mg pro Tag bestimmt ist.

3. Odiparcil zur Verwendung nach Anspruch 1 oder Anspruch 2, das zur oralen Verabreichung bestimmt ist.

4. Odiparcil zur Verwendung nach Anspruch 3, das zur Verabreichung mit der Nahrung bestimmt ist.

5. Odiparcil zur Verwendung nach einem der Ansprüche 1 bis 4 bei der Behandlung des Maroteaux-Lamy Syndroms.

6. Pharmazeutische Zusammensetzung, die Odiparcil und ein oder mehrere pharmazeutisch verträgliche Trägerstoffe enthält, zur Verwendung bei der Behandlung der Mucopolysaccharidose des Typs III, VI oder VII.

7. Pharmazeutische Zusammensetzung zur Verwendung nach Anspruch 6, die von 100 mg bis 1000 mg Odiparcil enthält.

8. Pharmazeutische Zusammensetzung zur Verwendung nach Anspruch 6 oder Anspruch 7, die in oraler galenischer Form vorliegt, vorzugsweise einer festen Formulierung.

9. Pharmazeutische Zusammensetzung zur Verwendung nach Anspruch 8, die eine Tablette darstellt.

10. Pharmazeutische Zusammensetzung zur Verwendung nach einem der Ansprüche 6 bis 9 bei der Behandlung des Maroteaux-Lamy Syndroms.

## Claims

1. Odiparcil for use in the treatment of a mucopolysaccharidosis of type III, VI or VII.

2. Odiparcil for the use of claim 1, which is intended to be administered at a daily dose of about 100 mg to about 5000 mg.

3. Odiparcil for the use of claim 1 or claim 2, which is intended to be administered orally.

4. Odiparcil for the use of claim 3, which is intended to be administered with food.

5. Odiparcil for the use of one of claims 1 to 4 in the treatment of Maroteaux-Lamy disease.

6. A pharmaceutical composition containing odiparcil and one or several pharmaceutically acceptable excipients, for use in the treatment of a mucopolysaccharidosis of type III, VI or VII.

7. The pharmaceutical composition for the use of claim 6, which contains from 100 mg to 1000 mg of odiparcil.

8. The pharmaceutical composition for the use of claim 6 or claim 7, which is an oral dosage form, preferably a solid form.

9. The pharmaceutical composition for the use of claim 8, which is a tablet.

10. The pharmaceutical composition for the use of one of claims 6 to 9 in the treatment of Maroteaux-Lamy disease.
